Europäisches Patentamt

European Patent Office

Office européen des brevets

(19)

(11) Publication number: **0 020 001**
A1

(12)

# EUROPEAN PATENT APPLICATION

(21) Application number: **80301180.8**

(22) Date of filing: **14.04.80**

(51) Int. Cl.³: **B 44 C 5/04**, B 32 B 29/00

(30) Priority: **21.05.79 US 41270**

(43) Date of publication of application: **10.12.80**
Bulletin 80/25

(84) Designated Contracting States: **BE CH DE FR GB IT LI NL SE**

(71) Applicant: FORMICA CORPORATION, Berdan Avenue, Wayne, New Jersey06904 (US)

(72) Inventor: **West, Wilbur Winton, 7752 Bitteroot Lane, Cincinnati, Ohio (US)**
Inventor: **Schiermeier, Walter William, 3681 Riehle Road, Cincinnati, Ohio (US)**

(74) Representative: **Aliam, Peter Clerk et al, LLOYD WISE, TREGEAR & CO. Norman House 105-109 Strand, London WC2R 0AE (GB)**

(54) **Registered embossed laminates and method for producing same.**

(57) The invention provides a heat and pressure consolidated laminate of registered embossment and color contrast consisting essentially of, in superimposed relationship:

(a) a core layer, said core layer consisting of a self-supporting substrate;

(b) a kraft paper sheet impregnated with a first thermoset resin; and

(c) a surface coating, said surface coating consisting of a pigmented, second thermoset resin positioned above the kraft paper sheet, the surface of said laminate containing an embossment consisting of protuberances and valleys of contrasting colors, the color of said kraft paper sheet being primarily evidenced at the valleys thereof and the color of said second resin being primarily evidenced at the protuberances thereof.

The invention enables the conventionally used decor and overlay sheets of expensive alphacellulose paper to be avoided.

ACTORUM AG

REGISTERED EMBOSSED LAMINATES AND METHOD
FOR PRODUCING SAME

This invention generally relates to decorative laminates and methods of producing the same.  More particularly, this invention relates to a registered embossed laminate of color contrast employing a melamine-formaldehyde saturated kraft sheet coated with a pigmented melamine-formaldehyde resin as a replacement for standard decor and overlay sheets.

Conventionally, decorative laminates are made of three essential layers:  a core layer, a print layer, and a surface layer.  The core layer constitutes a bottom or supporting layer onto which the other layers are bonded.  In normal high-pressure laminate manufacture the core layer consists of a plurality of cellulosic sheets.  The core sheets are generally made from a kraft paper impregnated with a laminating resin.  Laminating resins commonly used for the core layer include phenolic, amino, epoxy, polyester, silicone, and diallyl phthalate resins to name but a few.  The industrially preferred laminating resin for decorative laminates is a phenolic resin made from the reaction of phenols with formaldehyde.  In low-pressure laminate manufacture, the core layer is generally comprised of a sheet of particleboard ranging from 3/8" to 1" in thickness.

Placed above the core layer is the print layer which is generally an alpha cellulose pigmented paper containing a print, pattern, or design that has been impregnated with a melamine-formaldehyde resin.  Typically, the printing is performed prior to impregnation by a high-speed rotogravure.

The cured melamine-formaldehyde resins are colorless and resistant to light; they are resistant to a variety of solvents and stains; and their heat resistance makes them immune to burning cigarettes, boiling water and heated containers up to about 325°F. Without these melamine-formaldehyde resins, the decorative laminate industry would not exist as it is known today. However, because these resins are extremely brittle, they sometimes require reinforcement.

The surface layer, or overlay as it is commonly referred to, is a high-quality alpha cellulose paper impregnated with a melamine-formaldehyde resin. This layer protects the print sheet from external abuse such as abrasive wear and tear, harsh chemicals, burns, spills and the like. It is primarily the melamine-formaldehyde resin which accounts for these protective properties. The alpha-cellulose paper acts as a translucent carrier for water-thin resin, imparts strength to the rather brittle melamine-formaldehyde resin, maintains a uniform resin thickness in the overlay by acting as a shim, and controls resin flow.

Typically, the core layer, print layer and surface layer are stacked in a superimposed relationship, between polished steel plates and subjected to a pressure and temperature for a time sufficiently long enough to cure the laminating resins impregnating the respective layers. The elevated temperatures and pressure actually cause the impregnated resins within the sheets to flow which consolidates the whole into an integral mass, known as the laminate. These laminates find use as counter tops, table tops, furniture, store fixtures and the like.

Employment of the alpha cellulose decor and overlay sheets have long constituted major problems to the decorative laminate industry. These highly refined cellulosic papers are the result of complex and expensive pulp processing. Their availability is often as limited as their cost is high. In today's environment of ever diminishing natural resources and escalating prices, the decorative laminate industry has been eagerly searching for viable substitutes to replace the alpha cellulose sheets.

The major obstacle preventing kraft papers from being previously utilized in place of the alpha cellulose sheets is their inability to display the sharper, more intricate designs modern printing techniques have developed. Not only is the design clarity lost in the coarse fiber matte of the kraft sheet, but laminating resin show-through creates a visually disturbing appearance unacceptable in a decorative laminate.

## SUMMARY OF THE INVENTION

The present invention provides a color-contrasting, registered embossed laminate and a process for making the same, wherein in place of the alpha cellulose sheets hitherto used in the decor and overlay layers is substituted a melamine-formaldehyde saturated kraft paper sheet coated with a thickened and pigmented melamine-formaldehyde resin. The pigmented melamine-formaldehyde resin provides the decorative surface of the laminate.

## DETAILED DESCRIPTION OF THE LAMINATE

In accordance with the present invention, there is provided a heat and pressure consolidated laminate of registered embossment and color contrast consisting essentially of, in superimposed relationship, a core layer wherein said core layer consists of a self-supporting substrate, a kraft paper sheet impregnated with a first thermoset resin, and a surface coating wherein said surface coating consists of a pigmented, second thermoset resin positioned above the kraft paper sheet, the surface of said laminate containing an embossment consisting of protuberances and valleys each of which are of a color, the color of said kraft paper sheet being primarily evidenced at the valleys thereof and the color of said second resin being primarily evidenced at the protuberances thereof.

The heat and pressure consolidated laminate of the present invention is produced according to the method which comprises consolidating an assembly under heat and pressure wherein said assembly consists essentially of, in superimposed relationship, (1) a core layer comprising a self-supporting substrate, (2) a kraft paper sheet impregnated with a first thermosetting resin, (3) a surface coating wherein said surface coating consists of a pigmented, second thermosetting resin, said second thermosetting resin having a flow sufficient to cause said second thermosetting resin to flow more than said first thermosetting resin, and (4) an embossing press plate having a surface with protuberant and valley areas capable of being impressed into the uppermost surface of said kraft paper sheet and means for preventing the embossing press plate from sticking to the kraft paper sheet during lamination; said consolidating thereby effecting a lamination of the core layer, the kraft paper and the coating of said assembly together, an embossment of the uppermost of the kraft paper sheet and a migration of the pigmented, second thermosetting resin from the areas of said embossment corresponding to the protuberant areas of the embossing press plates to the areas of said embossment corresponding to the valley areas thereof and thereafter removing said embossing press plate from the resultant laminate so as to produce a dense laminate having embossed areas of contrasting color.

The core layer of the laminate may contain either a plurality of sheets impregnated with a laminating resin or a commercially available pressed particleboard. In high-pressure laminates, the core layer is typically provided by a plurality of impregnated sheets. These sheets can be varied in their nature in accordance with the particular properties desired in the decorative laminate. Typically, the core layer is made from paper, woven fabrics, mats, felts, or the like. Paper is by far the most widely used and thus constitutes the preferred stock for the core layer in high-pressure laminates. More particularly, a kraft paper of about a 60 to about a 130 pound basis weight per 3000 square foot ream is preferred as the stock from which the core layer sheets are prepared for high-pressure laminates because of its strong, cheap and plentiful nature. In low-pressure laminates, a particleboard of from about 3/8 to about 1 inch in thickness is preferred as the self-supporting substrate comprising the core layer. Suitable particleboards are commercially available in plentiful quantities at moderate cost.

The laminating resins used for impregnating the sheets of the core layer can be any of those thermosetting resins conventionally used in the production of laminates. Laminating resins conventionally used include, but are not limited to, phenolic, amino, epoxy, polyester, silicone and diallyl phthalate resins. The most commonly employed laminating resins, and that preferred in the instant invention, is the condensation product of a phenol and an aldehyde, generally referred to as a phenolic resin. In particular, it is preferable to employ a phenol-formaldehyde condensation product as the laminating resin employed in the core layer. These resins can be purchased commercially or prepared according to conventional procedures.

The kraft paper sheet employed over the core layer is substantially identical to the kraft paper sheets employed in certain embodiments of the core layer. More particularly, a kraft paper of about a 60 to about a 130 pound basis weight per 3000 square foot ream is preferred. The kraft paper sheet employed optionally may have displayed upon its surface a print, pattern, design or color suitable for the intended use of the laminate. The decorative print, pattern or design is typically applied by a high-speed rotogravure before the kraft sheet is impregnated with the melamine-formaldehyde resin. Photogravure reproductions of natural materials such as wood, marble, and leather may be applied to these kraft paper sheets as well. Typically, a three-ink system is employed with the kraft sheet itself being highly pigmented to provide a fourth color as background and as an opaque base by which the core layer is accordingly obscured. Although printing can be accomplished at any time prior to impregnation of the melamine-formaldehyde resin, printing is often done immediately before the impregnation.

The first thermosetting resin used to impregnate the topmost kraft paper sheet may be any thermosetting resin suitable for such purpose. For example, melamine-formaldehyde resins, melamine-urea-formaldehyde resins, unsaturated polyester resins and the like may be employed. If different resins are used in the impregnation of the kraft paper sheet from those in the coating layer, care should be taken to ensure that the resins are compatible, that is, the resins must be capable of forming strong coherent bonds with each other under the conditions used for consolidation and no deleterious reactions between the resins should occur. It is preferred to use only one type of resin for the purposes mentioned, and more preferably, to use a melamine-formaldehyde resin for these purposes.

Suitable melamine-formaldehyde resins are prepared by reacting melamine with formaldehyde in an aqueous dispersion or solution. The mole ratio of the melamine to formaldehyde may be varied from about 1:1 to about 1:3, respectively. It is preferred that the mole ratio be controlled from about 1:1.3 to about 1:2, melamine to formaldehyde, respectively. The reaction occurs under alkaline conditions at a temperature ranging from about 70° to the reflux temperature until a clear reaction product is obtained.

Various plasticizers and/or other modifiers may be incorporated into the impregnating resins in order to obtain improved properties during the curing of the material. Other modifiers may also be employed to improve release from press plates, workability and other properties of the amino plastic resins, as is known in the art.

Additionally, abrasive materials may be incorporated into the resin impregnated kraft paper sheet to increase abrasion resistance. Suitable abrasive materials include, but are not limited to, silica, silicates, glass fibers, clear asbestos, quartz, corundum, alumina and other suitable natural or synthetic abrasive materials.

The finely divided abrasive materials which lend to the laminate increased abrasion resistance may be incorporated into the topmost kraft paper sheet in various methods. For example, the abrasive material may be added in the pulper or in the head box during the paper-making process. This method causes uniform distribution of the abrasive particles throughout the sheet, the individual sheet fibers holding the particles through the formation of a matrix network around each particle. Additionally, the abrasive material may be added to the impregnating thermosetting resin. In this latter approach, a thin layer of abrasive particles is deposited on the surface of the kraft paper sheet during impregnation. In both instances the abrasive materials become an integral part of the sheet.

The pigmented, second thermosetting resin may comprise the same resin used in impregnating the kraft paper sheet or may be a different resin. The resins are different to the extent, however, that they should contain varying amounts of thinners, solvents, fillers, etc. so as to vary their viscosities and thereby cause the pigmented second thermosetting resin to flow more than the first thermosetting resin during the consolidation procedure.

The pigmented resin may be colored by the inclusion therein of any one of, or a combination of, inorganic or organic color pigments, extender pigments, metallic pigments, etc. The amount of pigment added to the resin will vary from 0.1% to 20%, based on the total weight of the resin, and dependent upon the pigment used and resultant color contrast desired. Typical inorganic pigments useful include those iron pigments ranging in color from yellow through red, reddish-brown, brown to black. Such iron pigments include yellow ocher, raw and burnt sienna, and raw and burnt umber. Other useful inorganic color pigments include chrome yellow, cadmium sulfide, zinc yellow, cobalt blue, ultramarine blue, iron oxide, chrome green, chromium oxide green, chromium hydroxide green, lamp black, and white pigments such as titanium dioxide, titanium calcium, zinc oxide, zinc sulfide, antimony oxide, lithopone, etc. Although lead pigments may be used, they are preferably avoided because of the safety hazard involved in their use. Organic pigments which may be used include toluidine red, phthalocyanine blue and green, Vandyke brown, alizarin, madder lake, lithol red, and the like.

Useful metallic pigments include aluminum powder, copper powder, bronze powders available in various shades depending upon the alloy composition, zinc powder, gold and gold-like powders, and the like. Any of the pigments, and particularly the metallic pigments, may be used alone or in combination with each other or in combination with other pigments. Furthermore, both resins may be pigmented as long as the colors employed are different and one flows more than the other, as discussed herein.

The second thermosetting resin may have incorporated into it any of the various plasticizers, modifiers and/or abrasive materials discussed above in relation to the first thermosetting resin. Additionally, when the second thermosetting resin is melamine-formaldehyde which may be modified through the addition of a fully hydrolyzed polyvinyl alcohol. If the second thermosetting resin produced is not viscous enough for coating purposes and penetrates the kraft paper sheet during the coating operation, a small amount of a thickening agent, such as carboxyl methyl cellulose, sodium alginate or the like may be added with the other constituents during the reaction to correct this result. Additionally, stabilizers such as o,p-toluene sulfonamide may be added to extend the shelf life of the resin coating.

The pigmented second thermosetting resin is coated upon the kraft paper sheet to a weight of from about 80 to about 150 grams per square meter, preferably about 100 to about 135 grams per square meter. The coating operation may be performed by a knife coater, reverse roll coater, or similar technique. The preferred coating process is the knife coater wherein the knife and resin dam are mounted on a roller, adjustment of the gap between the kraft paper sheet and the knife coater varies the coating thickness to its desired amount. The coating operation may be a separate operation or part of the impregnation operation. As a separate operation, the kraft paper sheet is first impregnated with the first thermosetting resin and subsequently dried before the coating operation is commenced. As part of the impregnation operation, coating is performed on the wet kraft paper sheet immediately subsequent to impregnation thereof and usually on a single apparatus. In the present invention, a combination of these two methods is preferred wherein the entire impregnation and coating processes are performed on a single apparatus which is provided with the means to impart a partial drying to the wet kraft paper sheet subsequent to impregnation thereof yet prior to coating, preferably by passing the impregnated kraft paper sheet through a hot air oven before the coating operation. After coating, the kraft paper sheet undergoes a terminal drying operation in which the coated print sheet is dried to a volatile content of from about 4% to about 9%, preferably about 6%.

The embossing press plates used in the novel process of the instant invention can be prepared by etching or machining a design on a metal plate. Alternatively, and more preferably, the plate can be prepared as disclosed in U.S. Patent No. 3,718,496, which patent is hereby incorporate by reference. Still further, the raised printed design technique, as taught by Grosheim in U.S. 3,373,068, will also work well as an embossing medium in the instant invention.

A preferred high-pressure laminate of the present invention is one in which 3 to 9 core sheets consisting of 6-20 ml. kraft paper have been impregnated with a 30% to 60% solution of phenol-formaldehyde resin so that the final resin solids content of the core sheets is about 20% to about 40% of the total weight of the core. Typically, these core sheets are oven dried after impregnation for a period of about one to two minutes at temperatures ranging from 140°C to 170°C. The kraft paper sheet consists of a 60 to 130 pound basis weight per 3,000 square foot ream kraft paper sheet, optionally displaying a print, pattern, design, or color, impregnated with a melamine-formaldehyde resin so that the final resin solids content of the kraft paper sheet is from about 10% to about 40% of the kraft paper sheet. The sheet is partially dried, coated on its print bearing side with a pigmented, melamine-formaldehyde resin and then terminally dried. The sheets are stacked such that the kraft paper sheet is the top most sheet with its print bearing and coated side facing outward. The stacked sheets are placed below an embossing press plate upon which there is exerted a pressure of from about 500 to 1600 psi at temperatures from about 120°C to 180°C for approximately 20 minutes to effect the cure and thereby provide the high-pressure laminate.

The embossed areas of the resultant laminate, i.e. those formed from the protuberances of the embossing plate and forming the valleys of the final embossment in the finished laminate surface should preferably be at least .007 inch deep. Depths of less than .007 inch are possible, but not preferred because the color contrast is not as evident. Laminates of muted color differentials can be produced using embossments of these lesser depths, however.

The release sheets used in the pressing of laminates of this invention, if necessary, are well known in the art and may be any non-adherable paper or non-adherable aluminum foil. The non-adherable paper may be a parchment type of paper of which a plurality are available commercially. The non-adherable metal foils, as well as the laminate of paper and the non-adherable foils, are also commercially available. Also suitable for this purpose are papers coated, treated or impregnated with polypropylene or the polyfluorocarbons and the like. Films of polypropylene may also be used. Silicone oil treated papers may be used and are also commercially available. Papers coated with sodium alginate and other salts of alginic acid are also suitable for this purpose and are available commercially. When "low pressure" laminates are being formed, no release sheet at all is necessary if the press plate has a releasing surface thereon. The same is true, but to a lesser degree, when one is producing "high pressure" laminates.

A preferred process for manufacturing a low-pressure laminate of the present invention is one in which a pressed particleboard of from about 3/8 to about 1 inch has stacked above it, in a manner identical to that employed in the high-pressure laminate, the coated kraft paper sheet. The coated kraft paper sheet is identical to the one employed in the high-pressure laminate. The stack is placed below an embossing press plate upon which there is exerted a pressure of from about 200 to about 600 psi at temperatures from 120°C to about 180°C for approximately 20 minutes to effect the cure and thereby provide the low-pressure laminate.

The following specific examples illustrate certain aspects of the present invention and, more particularly, point out methods of evaluating the unique advantages the decorative laminate of the present invention provides. However, the examples are set forth for illustration only and are not to be construed as limitations on the present invention except as set forth in the appended claims. All parts and percentages are by weight unless otherwise specified.

### EXAMPLE 1

A commercially available kraft paper having a basis weight of 94 pounds per 3,000 square foot ream is impregnated with a conventional phenol-formaldehyde laminating resin to a resin content of 32%. The impregnated paper is then dried to a volatile content of 6.0% and cut into sheets of suitable size. Six such sheets are assembled together to form the core layer.

A commercially available kraft paper having a basis weight of 115 pounds per 3,000 square foot ream is impregnated with a conventional melamine-formaldehyde resin to a resin content of 40%. Immediately thereafter the melamine-formaldehyde impregnated kraft paper sheet is coated with a pigmented, melamine-formaldehyde resin. The coating weight is 133 grams per square meter.

The pigmented, melamine-formaldehyde resin consists of a conventional melamine-formaldehyde resin in mixture with brown iron oxide, said brown iron oxide constituting 0.1% by weight of the melamine-formaldehyde resin. The pigmented resin has been thickened to a viscosity of 2000 cps with sodium alginate by adding thereto an amount approximately equal to 1.25% by weight of the resin.

The coated kraft paper sheet is dried to a volatile content of approximately 4 to 6%. The coated kraft paper sheet is placed over the core layer with its coated surface up. A tile textured embossing press plate is then placed over the coated kraft paper.

The entire assembly is subjected to pressure at 500 psi under 145°C for approximately 20 minutes. The resulting tile textured laminate has a reddish brown tile surface and a dark brown grout line. Excellent color registration is achieved. Approximately 95 to 100% of the pigmented coating resin has flowed out of the grout line thereby exposing the kraft paper sheet beneath the coating.

EXAMPLE 2

The process of Example 1 is followed in every material detail except that the melamine-formaldehyde impregnated kraft paper sheet is dried to a volatile content of about 6% before the pigmented resin coating is applied. The pigmented resin coating applied has a coating weight of approximately 95 grams per square meter, a viscosity of approximately 3500 cps and is pigmented using titanium dioxide in an amount equal to 15 weight percent of the resin. The resultant laminate exhibits an excellent color registration with approximately 95-100% of the pigmented coating resin having flowed out of the grout line.

EXAMPLE 3

The process of Example 1 is followed in every material detail except that the melamine-formaldehyde impregnated kraft paper sheet is partially dried before the pigmented resin coating is applied. The pigmented resin coating thereafter applied has a coating weight of approxi-

- 13 -

mately 115 grams per square meter, a viscosity of approximately 1500 cps and is pigmented using Vandyke brown in an amount equal to approximately 5 weight percent of the resin. The resultant laminate exhibits an excellent color registration with approximately 95-100% of the pigmented coating resin having flowed out ot the grout line.

## EXAMPLE 4

The process of Example 1 is followed in every material detail except that the core layer is now comprised of a pressed particleboard approximately 1/2 inch in thickness. The resultant laminate exhibits an excellent color registration with approximately the identical percent of the pigmented coating resin having flowed out of the grout line.

## EXAMPLES 5-7

The processes described in Examples 1-3 are followed in every material detail except that in each instance the kraft paper sheet is impregnated with a phenolic resin rather than the melamine-formaldehyde resin. The resulting laminates exhibit an embossed tile finish. Analysis shows that 40-60% of the coating resin has flowed out of the grout line. Color registration, although not as defined as in the previous examples, is none the less of a satisfactory nature.

<span>0020001</span>

CLAIMS:

1. A heat and pressure consolidated laminate of registered embossment and color contrast consisting essentially of, in superimposed relationship.

(a) a core layer, said core layer consisting of a self-supporting substrate;

(b) a kraft paper sheet impregnated with a first thermoset resin; and

(c) a surface coating, said surface coating consisting of a pigmented, second thermoset resin positioned above the kraft paper sheet, the surface of said laminate containing an embossment consisting of protuberances and valleys of contrasting colors, the color of said kraft paper sheet being primarily evidenced at the valleys thereof and the color of said second resin being primarily evidenced at the protuberances thereof.

2. The laminate of Claim 1 wherein the core layer comprises a plurality of kraft paper sheets impregnated with a laminating resin.

3. The laminate of Claim 2 wherein the laminating resin impregnating the core layer is a phenol-formaldehyde condensation product.

4. The laminate of any preceding claim wherein the core layer comprises pressed particleboard.

5. The laminate of any preceding claim wherein the first and second thermoset resins are melamine-formaldehyde resins.

6. The laminate of any preceding claim wherein the surface coating has a coating weight of about 80 to 150 grams per square meter, a viscosity from about 1500 to 4000 cps and the pigment constitutes from about 0.1 to 20 weight percent of the second thermoset resin.

7.  The laminate of Claim 6 wherein the coating weight is about 135 grams per square meter, the viscosity is about 2000 cps and the pigment constitutes about 0.5 weight percent of the second thermoset resin.

8.  The laminate of any preceding claim wherein the first and second thermoset resins are both melamine--formaldehyde resins.

9.  A method for producing a heat and pressure consolidated laminate which comprises consolidating an assembly under heat and pressure wherein said assembly consists essentially of, in superimposed relationship:

(i)   a core layer, said core layer consisting of a self-supporting substrate.

(ii)   a kraft paper sheet impregnated with a first thermosetting resin,

(iii)  a surface coating, said surface coating consisting of a pigmented, second thermosetting resin, said second thermosetting resin having a flow sufficient to cause said second thermosetting resin to flow more than said first thermosetting resin, and

(iv)   an embossing press plate having a surface with protuberant and valley areas capable of being impressed into the uppermost surface of said kraft paper sheet and means for preventing the embossing press plate from sticking to the kraft paper sheet during lamination; said consolidating thereby effecting a lamination of said assembly together, an embossment of the uppermost of the kraft paper sheet and a migration of the pigmented, second thermosetting resin from the areas of said embossment corresponding to the protuberant areas of the embossed press plates to the valley areas thereof and thereafter removing said embossing press plate from the resultant laminate so as to produce a dense laminate having embossed areas of contrasting color.

LLOYD WISE, TREGEAR & CO.
NORMAN HOUSE 105-109 STRAND

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| X | US - A - 4 092 198 (H.J. SCHER et al.)<br>* Entire document *<br>-- | 1-3,5, 6,8,9 | B 44 C 5/04<br>B 32 B 29/00 |
| E | EP - A - 0 009 813 (RESOPAL)<br>* Entire document *<br>-- | 1-4,9 | |
| A | US - A - 3 968 288 (R.F. TREXLER) | | |
| A | US - A - 3 802 947 (J.E. McQUADE) | | TECHNICAL FIELDS SEARCHED (Int.Cl. ³) |
| A | US - A - 3 666 604 (D. COFFET) | | B 44 C 5/04<br>3/08<br>- 1/24<br>A 47 B 13/08<br>B 32-B |
| A | US - A - 3 654 044 (H. HIROTA) | | |
| A | FR - A - 2 293 312 (EXXON) | | |
| A | FR - A - 2 030 513 (NISHIZAWA SHOJI CO LTD)<br>---- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-09-1980 | VAN THIELEN |